# EUROPÄISCHE PATENTSCHRIFT

(11) **EP 2 032 239 B1**
(45) Veröffentlichungstag und Bekanntmachung des Hinweises auf die Patenterteilung: **19.03.2014**
(21) Anmeldenummer: 07729319.9
(22) Anmeldetag: 21.05.2007
(51) Int. Cl.: B01D 71/02, B01D 67/00, B01D 53/22, B01D 61/36

(54) **VERFAHREN ZUR HERSTELLUNG EINER KOMPOSIT-MEMBRAN SOWIE VERWENDUNGEN**
PROCESS FOR PRODUCING A COMPOSITE MEMBRANE AND USES
PROCÉDÉ DE FABRICATION D'UNE MEMBRANE COMPOSITE ET UTILISATIONS

(30) Priorität: 13.06.2006 EP 06115334
(43) Veröffentlichungstag der Anmeldung: 11.03.2009
(73) Patentinhaber: BASF SE, 67056 Ludwigshafen (DE)
(72) Erfinder: DIEFENBACHER, Armin, 67361 Freisbach (DE); VOSS, Hartwig, 67227 Frankenthal (DE); SCHUCH, Gunter, 67071 Ludwigshafen (DE); NOACK, Manfred, 12683 Berlin (DE); VOIGT, Ingolf, 07743 Jena (DE); RICHTER, Hannes, 07629 Hermsdorf (DE); CARO, Jürgen, 13129 Berlin (DE)
(86) Internationale Anmeldenummer: PCT/EP2007/054875
(87) Internationale Veröffentlichungsnummer: WO 2007/144247

(56) Entgegenhaltungen:
- EP-A1- 0 481 660
- WO-A-00/20105
- DE-A1-102004 001 974
- US-A- 5 258 339
- US-A- 6 140 263
- US-B1- 6 177 373
- US-B1- 6 197 427

## Beschreibung

Die Erfindung betrifft ein Verfahren zur Herstellung einer Komposit-Membran gemäß Anspruch 1, welche mindestens einen porösen Träger und eine mikroporöse Trennschicht, die einen Zeolith vom MFI-Typ enthält, umfasst, eine Membran, erhältlich nach dem Verfahren, sowie deren Verwendung zur Gastrennung und Pervaporation.

Verfahren zur Herstellung von Komposit-Membranen sind bekannt. Die Herstellung solcher Membranen wird im Allgemeinen so durchgeführt, dass der Träger mit einer Lösung (Syntheselösung) in Kontakt gebracht wird, welche Komponenten enthält, aus denen in einer chemischen Reaktion (Synthese) die Trennschicht gebildet wird.

Dabei wird berichtet (z. B. in Poshuta et al., J. of Membrane Science 160 (1999), S. 115-125 oder Wong et al., J. of Membrane Science 193 (2001), S. 141-161), dass großer Wert auf die Erzeugung defektfreier Zeolithschichten zu legen ist, da Defekte einen unselektiven Fluss außerhalb der Zeolithporen nach sich ziehen.

Es wurde vorgeschlagen, diese Defektstellen beispielsweise durch eine zweite Membransynthese auf der Defekte enthaltenden Membran (z. B. Vroon et al., J. of Membrane Science 144 (1998), S. 65-76) oder durch nachbehandeln der Defekte in einem Sol-Gel-Verfahren (z. B. US 6,494,326) zu beseitigen.

Darüber hinaus leiten Kusakabe et al. in J. of Chem. Eng. of Jap. 30 (1997), S. 72-78 aus Permeationsmessungen mit verschiedenen Einzelgasen ab, dass nicht nur Defekte in der Zeolithschicht die Permeanz und die Permselektivität einer Membran beeinträchtigen, sondern auch die Verwachsung der Korngrenzen der Zeolithkristallite die Permeationseigenschaften beeinflusst. Es wird gefolgert, dass zur Steigerung der Selektivität Lücken an diesen Korngrenzen vermieden werden müssen. Es wird jedoch kein Weg aufgezeigt, wie dies erreicht werden kann.

Lin et al. berichten in Chem. Mater. 10 (1998) 3716-3723 über hohe n-Butan/Isobutan Selektivitäten von Membranen, die neben zeolithischen auch nichtzeolithische Poren aufweisen. Die Selektivität wird auf die geringe Größe oder Konzentration der nichtzeolithischen Poren zurückgeführt.

Nomura et al. beschreiben in J. of Membrane Science 187 (2001), S. 203-212, dass bei Zeolithmembranen neben einem Stofftransport durch die Zeolithporen (intrakristalliner Transportweg) auch ein Stofftransport zwischen den Zeolithkristalliten einer Membran auftreten kann (interkristalliner Transportweg). Sie zeigen außerdem an einer Silikalith-Membran durch Pervaporationsmessungen mit Ethanol/Wasser-Gemischen, dass auch diese interkristallinen Transportwege selektiv sein können. Es wird nicht beschrieben ob und wie man diese interkristallinen Transportwege beeinflussen kann.

Xomeritakis et al. beschreiben in Ind. Eng. Chem. Res. 40 (2001), S. 544-552, dass es bei der Trennung von o-Xylol und p-Xylol mit Membranen vom MFI-Typ neben dem Transport durch Zeolithporen und dem Transport durch meso- und makroporöse Defektstellen der Membran einen weiteren mikroporösen Transportweg, z. B. an den Korngrenzen, gibt. Es wird der Nachweis dieses Transportwegs durch Zugabe von n-Hexan zu einem Feedgemisch aus o-Xylol und p-Xylol anhand von Permeationsmessungen geführt. Ein Weg, die Bildung dieser mikroporösen Transportwege bereits bei der Membranherstellung zu beeinflussen, ist nicht beschrieben.

Es bestand daher die Aufgabe, ein Verfahren bereitzustellen, mit dem die Bildung mikroporöser, selektiven Transportwege in der Trennschicht MFI-haltiger Membranen beeinflusst und damit Selektivität und Durchlässigkeit verbessert werden kann.

Es wurde gefunden, dass die Aufgabe durch die Zugabe bestimmter Substanzen bei der Hydrothermalsynthese der Trennschicht gelöst werden kann.

Gegenstand der Erfindung ist daher ein Verfahren zur Herstellung einer Komposit-Membran gemäß Anspruch 1, wobei durch Hydrothermalsynthese auf einem porösen Träger eine oder mehrere, einen Zeolith vom MFI-Typ enthaltende mikroporöse Trennschichten erzeugt werden, dadurch gekennzeichnet, dass der Syntheselösung für die Hydrothermalsynthese ein oder mehrere Zuschläge aus der Gruppe lineare (C₁-C₄)-Alkohole, Ammoniak, primäre-, sekundäre und tertiäre Amine mit jeweils (C₁-C₄)-Alkylresten, ethanolamin und (C₃₋C₄)-Ketone zugegeben werden.

Durch Zugabe der erfindungsgemäßen Zusätze kann die Bildung mikroporöser Transportwege bei der Membranherstellung gezielt beeinflusst werden.

Bei Zugabe der erfindungsgemäßen Zusätze in geeigneten Mengen werden kleinere Kristallite gebildet, was zu einer Vergrößerung der Korngrenzfläche führt und die Transporteigenschaften an den Korngrenzen beeinflusst, ohne dass die Schichthaftung zu gering wird.

Die erfindungsgemäß hergestellten Membranen zeichnen sich durch erhöhte Durchlässigkeiten für die gewünschten Moleküle bei gleichzeitig guter Selektivität aus.

Gegenstand der Erfindung ist weiterhin eine Komposit-Membran, erhältlich nach dem oben beschriebenen Verfahren.

Weiterhin Gegenstand der Erfindung ist die Verwendung einer Komposit-Membran, erhältlich nach dem oben beschriebenen Verfahren, zur Stofftrennung durch Dampfpermeation, Gaspermeation oder Pervaporation und für die Filtration von Flüssigkeiten.

Der Begriff "mikroporös" wird im Sinne der IUPAC-Empfehlungen "Recommendations for the characterization of porous solids" Pure & Appl. Chem., 66 (1994) S. 1739-1758 verwendet. Mikroporös bedeutet damit, dass die Poren eine Weite von weniger als 2 nm haben.

Geeignete Träger sind Körper mit durchgängigen Poren mit Porendurchmessern zwischen 1 und 10 µm, die z. B. die Form von flachen Scheiben, Rohren oder Kapillaren aufweisen. Vorteilhaft ist auch die Form so genannter Multikanalelemente, die bei keramischen Membranen für die Mikro- oder Ultrafiltration Anwendung findet. Unabhängig von der geometrischen Form des Trägers ist ein so genannter asymmetrischer Aufbau des Trägers bevorzugt, bei dem dieser aus mehreren aufeinanderfolgenden Schichten mit abnehmendem Porendurchmesser besteht, wobei sich auf der mit der Trennschicht zu versehenden Seite des Trägers der kleinste Porendurchmesser befindet. Dieser beträgt bevorzugt 0,5 bis 100 nm, besonders bevorzugt 1 bis 60 nm. Als Material für den Träger eignet sich eine Vielzahl von Materialien wie z. B. Stähle oder oxidkeramische Materialien wie z. B. Aluminiumoxid, Titandioxid oder überwiegend aus Titandioxid bestehende Mischungen von Metalloxiden, geeignet sind aber auch Siliziumdioxid, Zirkoniumdioxid, Magnesiumoxid oder andere Metalloxide, sofern sie eine geringe Wasserlöslichkeit aufweisen.

Bevorzugt ist der Träger an den Stellen, an denen die aus ihm hervorgehende Membran mit einem Dichtungsmaterial in Berührung gebracht wird, mit einer geeigneten Hilfsschicht versehen, besonders bevorzugt einem an sich bekannten gasdichten und alkalibeständigen Glaslot.

Der genannte Träger wird zweckmäßigerweise vor den weiteren Arbeitsschritten gereinigt (z. B. durch Spülung mit sauren und/oder alkalischen wässrigen H₂O₂-Lösungen). Vorteilhaft ist auch ein nachfolgender Trocknungsschritt.

Die Hydrothermalsynthese wird bevorzugt in einer dem Fachmann an sich bekannten Weise durchgeführt.

Sie kann zum einen in der Weise erfolgen, dass sie ohne weitere Vorbehandlung direkt auf dem Träger erfolgt. Sie kann aber auch nach einem Seedingschritt (Impfschritt) erfolgen, bei dem auf der zu beschichtenden Seite des Trägers eine diese Seite ganz oder teilweise bedeckende Schicht von Impfpartikeln aufgebracht wird. Die Seedpartikel (Impfpartikel) können amorphe oder kristalline Körper mit Partikelgrößen zwischen 1 und 1000 nm sein, die in ihrer chemischen Zusammensetzung im wesentlichen der zu synthetisierenden Trennschicht entsprechen; in einer bevorzugten Variante bestehen diese aus MFI-Zeolith.

Die Herstellung der Seeds (Impfpartikel) erfolgt durch einen separaten hydrothermalen Prozess. Die Syntheselösung hat bevorzugt die folgende Zusammensetzung:
100 mol SiO₂ / 0 bis 1 mol Na₂O, bevorzugt 0 mol bis 0,4 mol Na₂O / 4 mol bis 40 mol TPAOH, bevorzugt 30 mol bis 40 mol TPAOH (Tetrapropylammoniumhydroxid) / 0 mol bis 36 mol TPABr (TPABr = Tetrapropylammoniumbromid), bevorzugt 0 bis 10 mol TPABr, wobei TPAOH + TPABr ≤ 36 mol sein sollte / 800 mol bis 10.000 mol H₂O : 400 mol bis 800 mol Ethanol, bevorzugt 400 mol Ethanol, wobei als SiO₂-Quelle TEOS (Tetraethylorthosilikat) verwendet wird.

Die genannten Tetrapropylammoniumsalze dienen als Struktur gebende Agenzien (Template). Es können stattdessen auch andere Template verwendet werden, z. B. 1,6-Hexandiol und Piperazin, eine ausführliche Beschreibung findet sich in: R. Szostak: Handbook of Molecular Sieves S. 521.

Die Lösung wird in einem geschlossenen Autoklavbehälter über 10 bis 500 Stunden zwischen 60°C und 100°C, bevorzugt bei 60°C bis 80°C langsam gerührt oder stehen gelassen. Die Weiterverarbeitung der Suspension erfolgt durch Verdünnen mit entionisiertem Wasser oder durch Abzentrifugieren des Feststoffes, mehrfaches Waschen und anschließendes Redispergieren in entionisiertem Wasser, das mit NaOH auf pH 10 bis pH 12 oder mit NH₃ oder Wasser auf pH 7-10 eingestellt worden ist.

Die Aufbringung der Seedpartikel auf den Träger kann auf verschiedene Arten erfolgen: Zum einen kann dies durch Auffiltrieren (slip casting) geschehen, d. h. eine die Seedpartikel enthaltende, vorzugsweise wässrige, Lösung wird mit der zu beschichtenden Seite des Trägers in Kontakt gebracht und, entweder infolge von Anlegen eines Überdrucks auf der zu beschichtenden Seite gegenüber der nicht zu beschichtenden Seite, oder infolge eines von der Poren ausgeübten Kapillarsogs, wird die die Seedpartikel umgebende Lösung in die Poren eingetragen, während die Seedpartikel sich, sofern sie größer als die Poren sind, auf der zu beschichtenden Seite des Trägers akkumulieren.

Zum anderen kann das Anheften der Seedpartikel an den Träger aber auch durch ein geeignetes Hilfsmittel erfolgen. Hier eignen sich insbesondere mono- oder polymere quaternäre Ammoniumsalze, wie z. B. Poly-DADMAC (Redifloc®),(DADMAC = Diallyldimethylammoniumchlorid).

Als Syntheselösungen für die Hydrothermalsynthese (im folgenden kurz "Synthese" genannt) werden im Allgemeinen dem Fachmann an sich bekannte Mischungen eingesetzt. Bevorzugt sind zum einen Mischungen, die sich zur Bildung eines MFI-Zeolithen mit einem hohen Siliziumanteil (high silica zeolith), d. h. von 20 < SiO₂-Al₂O₃ < ∞ eignen. Bevorzugt ist insbesondere Silikalith.

Die Herstellung solcher Zeolithe ist in der WO-A 2005/068057 beschrieben, auf die hiermit ausdrücklich Bezug genommen wird und die durch Zitat als Bestandteil dieser Beschreibung gilt.

Bevorzugt sind auch Zeolithe mit einem höheren Aluminiumanteil, insbesondere ZSM 5.

Die Syntheselösungen enthalten die folgenden molaren Bestandteile, wobei A den erfindungsgemäßen Zuschlag bedeutet:
SiO₂/Al₂O₃/Na₂O/TPAOH/TPABr/H₂O/A = 1/5x10-5 bis 5x10-2, bevorzugt 6x10-5 bis 1x10-2 / 0 bis 0,2 bevorzugt 0,005 bis 0,02 / 0 bis 0,1, bevorzugt 0,03 bis 0,08 / 0 bis 0,1, bevorzugt 0,03 bis 0,08 / (10-y) bis (100-y), bevorzugt (20-y) bis (50-y) / y mit 0 < y ≤ 50.

Gegebenenfalls kann die Syntheselösung auch Isopropanol enthalten.

Bevorzugte erfindungsgemäße Zuschläge A sind von den Alkoholen Methanol, Ethanol, n-Propanol und n-Butanol, besonders bevorzugt Methanol und Ethanol. Von den Aminen bevorzugt sind primäre und sekundäre, besonders bevorzugt primäre, Amine. Die Alkylreste an den Aminen sind bevorzugt Methyl- und Ethyl- und n-Propylgruppen. Besonders bevorzugt ist n-Propylamin. Ebenso bevorzugt ist Ammoniak. Als Keton ist Aceton bevorzugt. Insbesondere bevorzugte Zuschläge A sind Methanol, Ethanol, n-Propanol und n-Propylamin. Es können auch Mischungen von zwei oder mehr erfindungsgemäßen Zuschlägen eingesetzt werden.

Der molare Anteil des Zuschlags A in der Syntheselösung y beträgt bevorzugt 15-35, besonders bevorzugt 20-35, und ganz besonders bevorzugt 25-35.

Das molare Verhältnis von H₂O und A in der Syntheselösung beträgt 100-1 : 1, vorzugsweise 10 - 2 : 1, besonders bevorzugt 5 - 2 : 1.

Die genannten Tetrapropylammoniumsalze dienen als Struktur gebende Agenzien (Template). Es können stattdessen auch andere Template verwendet werden, z. B. 1,6-Hexandiol und Piperazin, eine ausführliche Beschreibung findet sich beispielsweise in: R. Szostak: Handbook of Molecular Sieves S. 521.

Die SiO₂-Quelle ist im Allgemeinen ein kolloidales Kieselsol wie z. B. Levasil (Fa. H.C. Starck) oder eine siliziumorganische Verbindung wie z. B. TEOS (Tetraethylorthosilikat).

Im Rahmen der Erfindung bedeuten die Begriffe "Zugabe und "zugeben" im Zusammenhang mit den erfindungsgemäßen Zuschlägen A eine Zugeben des Zuschlags in freier Form zu der Syntheselösung.

Bei Verwendung von beispielsweise siliziumorganischen Verbindungen als SiO₂-Quelle kann bereits im Syntheseansatz eine Verbindung A gebunden in stöchiometrischen Mengen enthalten sein, die im Laufe der Synthese freigesetzt wird. Zum Beispiel werden bei Verwendung von TEOS vier Äquivalente Ethanol freigesetzt, was einem y-Wert von 4 entspricht.

Auch in diesem Fall kann durch das erfindungsgemäße Verfahren der Transport durch interkristalline Transportwege gezielt beeinflusst werden.

Dies kann durch Zugabe des entsprechenden Zuschlags in den Syntheseansatz oder durch Abziehen des entsprechenden Zuschlags aus dem Syntheseansatz erfolgen. Beispielsweise ergeben sich bei Verwendung von TEOS als SiO₂-Quelle bei Zugabe von Ethanol Werte von y > 4 und durch Abziehen von Ethanol z. B. durch Vakuumrotationsverdampfung Werte von y < 4.

Als Al₂O₃-Quelle kann gegebenenfalls das im genannten Silikat enthaltene Aluminium oder eine aluminiumorganische Verbindung wie z. B. Aluminiumisopropylat oder ein lösliches Al-Salz wie Al-Chlorid, -Nitrat,-Sulfat dienen.

Das verwendete Wasser ist bevorzugt ein mittels Ionentauscher entsalztes Wasser, besonders bevorzugt ein mittels Ionentauscher mit nachfolgender mindestens einmaliger Destillation entsalztes Wasser.

Die Syntheselösung wird beispielsweise so hergestellt, dass Wasser, der erfindungsgemäße Zuschlag, TPAOH, TPABr und gegebenenfalls die Aluminiumquelle vorgemischt und für 1 bis 120 min, bevorzugt 5 bis 60 min gerührt wird und anschließend die SiO₂-Quelle in gelöster, kolloidal gelöster oder suspendierter Form innerhalb von 1 bis 100 min, bevorzugt 2 bis 50 min eingetragen wird. Bei Verwendung von TEOS als Si-Quelle erfolgen erst seine Hydrolyse mit Wasser und einer Teilmenge des Templats und dann gegebenenfalls die Vereinigung mit der Al-Quelle, dem Alkali, dem Resttemplat und dem Restwasser.

Die Lösung wird dann weitere 1 bis 200 min, bevorzugt 5 bis 100 min gerührt und 1 bis 150 min, bevorzugt 5 bis 50 min ohne Rühren gealtert.
Während der vorgenannten Schritte wird die Temperatur bei 5 bis 100, bevorzugt 15 bis 40 °C, gehalten.

Ein erfindungsgemäßer Syntheseansatz kann auch so hergestellt werden, dass Wasser, TPAOH, TPABr und gegebenenfalls die Aluminiumquelle vorgemischt und für 1 bis 120 min, bevorzugt 5 bis 60 min gerührt wird und anschließend die SiO₂-Quelle in gelöster, kolloidal gelöster oder suspendierter Form innerhalb von 1 bis 100 min, bevorzugt 2 bis 50 min eingetragen wird.

Die Lösung wird dann weitere 1 bis 200 min, bevorzugt 5 bis 100 min gerührt und 1 bis 150 min, bevorzugt 5 bis 50 min ohne Rühren gealtert.

Erst nach erfolgter Hydrolyse wird der erfindungsgemäße Zuschlag zum Syntheseansatz gegeben oder, wenn eine entsprechende Verbindung bei Verwendung einer siliziumorganischen Verbindung als SiO₂-Quelle durch die Hydrolyse in stöchiometrischen Mengen entsteht, können kleinere Mengen als die durch die SiO₂-Quelle stöchiometrisch entstandene Menge durch Abziehen der Verbindung eingestellt werden.

Während der genannten Schritte wird die Temperatur bei 5 bis 100, bevorzugt 15 bis 40 °C, gehalten.

Es schließt sich die eigentliche Synthese an, bei der die Lösung über eine Dauer von 1 bis 100 h, bevorzugt 5 bis 50 h, besonders bevorzugt 10 bis 20 h, mit dem geimpften Träger in Kontakt gebracht wird, wobei die Synthesetemperatur 100 bis 250 °C, bevorzugt 140 bis 210 °C, besonders bevorzugt 170 bis 190 Grad beträgt. Das in Kontakt Bringen kann auf verschiedene Weisen erfolgen: so kann während der Synthesezeit die Syntheselösung im Wesentlichen ruhen oder sie kann stetig oder in regelmäßigen oder unregelmäßigen Abständen über den zu beschichtenden Träger in gleicher oder wechselnder Richtung bewegt werden. Vorteilhaft ist dabei eine Verfahrensweise, welche dafür sorgt, dass die Syntheselösung überwiegend mit den zu beschichtenden Oberflächen des Trägers in Berührung kommt und weniger mit der entgegengesetzten Seite. Ist z. B. im Falle einer Rohrmembran eine innenseitige Beschichtung erwünscht, so ist es vorteilhaft, den Kontakt der Syntheselösung mit der Rohraußenseite zu erschweren. Dies kann zum einen erreicht werden, indem die nicht zu beschichtende(n) Oberfläche(n) in geeigneter Weise durch eine entfernbare und für die Syntheselösung schwer zu durchdringende Schicht abgedeckt wird. Im Falle des innenseitig zu beschichtenden Rohres kann diese abdeckende Schicht eine Umwicklung mit einem Band, z. B. aus PTFE sein oder eine geeignete aufstreichbare Polymerlösung. Zum anderen kann der Zutritt der Syntheselösung zu den nicht zu beschichtende(n) Oberfläche(n) aber auch dadurch erschwert werden, dass die Poren des Trägers während der Synthese mit einem Medium gefüllt sind, das den Durchtritt der Syntheselösung durch die Poren des Trägers erschwert bzw. verhindert wie dies in der WO 2005/068056 A1 beschrieben ist. Bei diesem Medium kann es sich handeln um
- eine Flüssigkeit in der die Syntheselösung nur wenig löslich ist oder
- einen Feststoff, der als Schmelze in die Poren des Trägers eingebracht wird und nach Beendigung der Synthese durch Schmelzen oder Lösen mit einem geeigneten Lösungsmittel entfernt wird oder
- ein Gas, z. B. Luft oder Stickstoff, das sich in dem an die nicht zu beschichtende(n) Oberfläche(n) angrenzenden Raum und zumindest teilweise in den Poren des Trägers befindet, wobei der Druck des Gases so eingestellt wird, dass ein Durchtreten der Syntheselösung von der zu beschichtenden Seite auf die nicht zu beschichtende Seite des Trägers unterbunden wird.

Es ist vorteilhaft, die Präparation der seeds und der Syntheselösung sowie die Hydrothermalsynthese selbst in Gefäßen durchzuführen, die praktisch kein Aluminium an die Lösungen abgeben können. Gesignet sind insbesondere Alarme Stähle und/oder organische Polymere wie z.B. PTFE, PFA, (PTFE = Polytetrafluorethylen, PFA = Perfluoralkoxy-Copolymere), Polypropylen oder Werketoffen, die auf den von Lösung berühten Flächen mit mindestens einem der genannten Materialien beschichtet sind.

Nach der Synthese ist zwecks Entfernung von Alkalispuren ein ein- oder mehrmaliges Spülen mit Wasser oder einer sauren Lösung vorteilhaft. Bei der sauren Lösung kann es sich um wässrige Lösungen von anorganischen oder organischen Säuren, wie Essig- oder Ameisensäure, handeln, wobei die Säurekonzentration 10⁻⁵ bis 1 mol/l, bevorzugt 10⁻⁴ bis 0,01 mol/l beträgt und die Dauer der Spülungen 5 bis 120 min, bevorzugt 10 bis 90 min beträgt.

Danach erfolgt im Allgemeinen eine Trocknung bei einer Temperatur von 5 bis 40 °C für 1 bis 100 h, bevorzugt 10 bis 30 h, wobei sich über dem Trocknungsgut ein strömendes oder ruhendes Gas, bevorzugt Stickstoff oder Luft, befindet.

Nach der Trocknung wird die Membran im Allgemeinen kalziniert, indem mit einer Aufheizrate von 0,1 bis 1 K/min aufgeheizt wird bis zu einer Temperatur von 200 bis 600 Grad, bevorzugt 350 bis 500 °C, wobei bei einer Zwischentemperatur von 300 bis 400 °C eine Haltezeit von 100 bis 500 min vorteilhaft sein kann.

Bei der Endtemperatur wird 30 min bis 300 min verweilt, danach wird mit einer Rate von 0,1 K/min bis 10 K/min abgekühlt.

Die so erzeugte Membran kann noch einem weiterem Behandlungsschritt unterzogen werden, bei dem zwecks Erzeugung einer geschlossenen Zeolithschicht mindestens eine weitere Schicht aufgebracht wird.

Die Membran kommt bevorzugt in Modulen zum Einsatz, in denen jeweils mindestens eine der beschriebenen Membranen so eingedichtet ist, dass die MFI-Schicht den Feed- und den Permeatraum voneinander trennt. Die Eindichtung kann, sofern die Membranen in Form von Rohren oder Multikanalelementen vorliegen, mittels O-Ringen aus Elastomeren oder durch Eingießen der Elemente in eine polymere oder keramische Vergussmasse an mindestens einem Ende der Elemente und nachfolgendes Abschneiden der Vergussmasse getätigt werden. Das Eingießen an lediglich einem Ende ist sinnvoll im Falle von Rohrmodulen, bei denen der Feedraum sich auf der Rohraußenseite befindet, und bei denen die Rohre am nicht eingedichteten Ende verschlossen sind.

Bevorzugt sind eines oder mehrere der beschriebenen Module Bestandteil(e) einer Membraneinheit. Diese kann auf mehrere dem Fachmann an sich bekannten Arten betrieben werden: entweder zur Gasseparation, bei der der Feedstrom gasförmig mit der Membran in Kontakt gebracht wird, oder zur Pervaporation, wobei das zu trennende Gemisch (Feed) in flüssiger Form mit der Membran in Kontakt gebracht und der die Membran passierende Strom (Permeat) gasförmig abgezogen wird. Die Temperatur, bei der das zu trennende Gemisch mit der Membran in Kontakt gebracht wird, liegt im Allgemeinen zwischen 20 und 300 °C, bevorzugt 50 bis 200 °C. Der Druck beträgt auf der Feedseite der Membran im Allgemeinen 1 bis 100, bevorzugt 1 bis 35 bar abs. Der Druck beträgt auf der Permeatseite 1 bis 20000, bevorzugt 10 bis 10000 mbar abs., wobei der Druck auf der Feedseite im Allgemeinen höher als auf der Permeatseite ist. Der permeatseitige Druck wird eingestellt durch Abführen des Permeatstroms mittels einer Vakuumpumpe und/oder eines Kompressors und/oder durch Kondensieren des Permeatstroms bei einer Temperatur, die zu einem dem gewünschten Permeatdruck entsprechenden Eigendruck des Permeatgemischs führt. Es ist aber auch möglich, den Partialdruck der permeierenden Komponenten zu senken durch Einführen eines Spülgases ("Sweepgas") auf die Permeatseite. Als Sweepgase sind z. B. Stickstoff oder Wasserdampf geeignet.

Im Falle der Pervaporation kann es vorteilhaft sein, die benötigte Membranfläche auf mehrere Apparate aufzuteilen, und, zwecks Ausgleich des durch den Phasenübergang flüssig-gasförmig verursachten Wärmeverlusts, einen oder mehrere Wärmeübertrager zwischen die Membranapparate zu schalten.

Die Membraneinheit kann aber auch in der dem Fachmann an sich bekannten Art einer Dampfpermeation betrieben werden, die sich von der Pervaporation dadurch unterscheidet, dass der Feed dampfförmig mit der Membran in Kontakt gebracht wird.

Das Membranverfahren kann zum einen einstufig ausgeführt sein, d. h. sowohl das Retentat als auch das Permeat aus einem Membranapparat oder die vereinigten Permeate aus mehreren vom Feed hintereinander und/oder parallel durchströmten Membranapparaten verlassen ohne weitere Behandlung die Membraneinheit. Das Membranverfahren kann aber auch zwei- oder mehrstufig ausgeführt sein, wobei aus einer Stufe das Permeat als Feed in die jeweils folgende Stufe geführt wird und das Retentat aus dieser Stufe dem Feed in die erstgenannte Stufe zugemischt wird. Derartige Anordnungen sind an sich bekannt (siehe z. B. Sep.Sci.Technol. 31 (1996), 729 ff).

Die erfindungsgemäßen Komposit-Membranen eignen sich zur Stofftrennung durch Dampfpermeation, Gaspermeation oder Pervaporation und für die Filtration von Flüssigkeiten.

Bevorzugt eignen sich die Komposit-Membranen zur Trennung von Kohlenwasserstoffgemischen, besonders bevorzugt von isomeren linearen und verzweigten Kohlenwasserstoffen, insbesondere n-Butan und iso-Butan, bzw. n- und iso-Buten.

Durch Pervaporation können Flüssigkeiten mit unterschiedlichen Polaritäten aufgetrennt werden, beispielsweise Alkohole und Wasser, insbesondere Ethanol/Wasser - oder Isopropanol/Wasser Gemische.

Die Erfindung wird an den nachfolgenden Beispielen erläutert:

### Beispiel 1:

### a) Vorbehandlung der Träger:

Drei poröse Träger in Rohrform (Länge 250 mm, Außendurchmesser 10 mm, Innendurchmesser 6 mm, Porengröße innen 5 nm, an den Enden versehen mit Glaslot) aus TiO₂ wurden zunächst mit Seeds (Silikalith-Kristalle mit einer Größe von 30 - 100 nm) innen mittels Auffiltrieren. Danach wurde das Rohr mit einer Rate von 0,75 K/h auf 400 °C aufgeheizt, 7 h bei 400 °C gehalten und dann mit einer Rate von 0,75 K/h auf Raumtemperatur abgekühlt. Danach wurde das Rohr außenseitig mit PTFE-Band umwickelt und in eine gemäß nachfolgender Beschreibung hergestellte Syntheselösung gestellt.

### b) Herstellung der Syntheselösung:

Die Zusammensetzung der Syntheselösungen ist in der Tabelle angegeben. Die Quelle für SiO₂, Al₂O₃ und Na₂O war das Kieselsol Levasil® 300/30% (Fa. Kurt Obermeier, Bad Berleburg, Deutschland) mit einem Verhältnis SiO₂/Al₂O₃/Na₂O von 90/0,15/1,66. Wasser (gereinigt über Ionentausch und zweifache Destillation), TPAOH (40 %ige wässrige Lösung, Fa. Alfa Aesar), TPABr (Fa. Merck) sowie der erfindungsgemäße Zuschlag wie in der Tabelle angegeben wurden in einen Erlenmeyerkolben aus Polypropylen gegeben und bei Raumtemperatur 30 min gerührt. Anschließend wurde das Levasil unter Rühren zugetropft.

| Membran | Molverhältnis in der Syntheselösung ¹⁾ | Art des Zuschlags |
|---|---|---|
| 1 | 90/0,225/1/4,15/1,85/1990/0 | ohne Zuschlag |
| 2 | 90/0,225/1/4,15/1,85/1490/500 | Methanol |
| 3 | 90/0,225/1/4,15/1,85/1490/500 | Ethanol |
| 4 | 90/0,225/1/4,15/1,85/1490/500 | n-Propanol |
| 5 | 90/0,225/1/4,15/1,85/1490/500 | Ethanolamin |
| 6 | 90/0,225/1/4,15/1,85/1490/500 | n-Propylamin |
| 7 | 90/0,225/1/4,15/1,85/1790/200 | Ethanol |
| 8 | 90/0,225/1/4,15/1,85/1640/350 | Ethanol |
| 9 | 90/0,225/1/4,15/1,85/1390/600 | Ethanol |
| ¹⁾SiO₂/Al₂O₃/Na₂O/TPAOH/TPABr/H₂O/Zuschlag | | |

### Hydrothermalsynthese:

Die Synthesen erfolgten bei einer Temperatur von 180 °C über eine Dauer von 24 h, indem der kalte Autoklav mit der Syntheselösung und dem geseedeten, außen mit Teflonband bewickelten Träger in einen vorgeheizten Trockenschrank gestellt wurde. Nach der Synthese wurde das Teflonband entfernt.

### c) Nachbehandlung:

Die Membran wurde in einen Messzylinder gestellt und unter Rühren 4 mal wechselweise mit 0,1 m Ameisensäure und Wasser jeweils 30 min gewaschen. Anschließend wurden die Membranen ca. 16 h an der Raumluft trocknen gelassen und danach in einen Umluftofen gegeben, dort mit einer Rate von ca. 0,3 K/h zunächst auf 450 °C aufgeheizt und dort für 400 min belassen. Anschließend wurde mit einer Rate von 17 K/h auf Raumtemperatur abgekühlt.

### d) Permeationsversuche:

Für die Permeationsversuche wurden die Membranen in einem Testmodul platziert. Die Abdichtung des Feedraums gegen den Permeatraum wurde mittels einer O-RingDichtung erreicht. Dabei wurden die O-Ringe auf die verglasten Enden des Trägers gesteckt.

Das Testmodul wurde in einem Ofen platziert.

Vor den Messungen wurden die Membranen evakuiert und die Zuführungsleitungen und der Ofen auf 130°C vorgeheizt. Danach wurden die Einzelgasflüsse von H₂, N₂, 1-Buten und i-Buten bestimmt. Nach jeder Einzelgasmessung wurde der Feed- und Permeatraum evakuiert.

Das Testgas war eine 50/50-Mischung 1-Buten/Isobuten (Fa. Linde, Reinheit der Gase jeweils 99.5 %), vorliegend in einem Gaszylinder. Aus diesem wurde das Testmodul feedseitig angeströmt. Nach dem Testmodul wurde der feedseitige Druck mittels eines Hinterdruckreglers auf 2,5 bar abs. eingeregelt. Die Messung der Permeat- und Retentatmenge erfolgte über kommerziell erhältliche Seifenblasenzähler.

Während der Permeationsmessungen wurde die Temperatur des Testmoduls bei 130°C gehalten.

Der das Testmodul verlassende Permeatstrom (Permeatdruck: ca. 1 bar abs.) wurde in die Probennahme-Schleife eines GC-MS-Geräts geleitet und dort analysiert.

Die Ergebnisse der Messungen sind in der nachfolgenden Tabelle dargestellt (1-Buten Permeanz J_{1-Buten} und Permselektivität PS). Dabei ist die Permeanz einer Komponente i die lokale Permeatflussdichte der Komponente i geteilt durch die Partialdruckdifferenz zwischen Feed- und Permeatseite der Komponente i. Die Permselektivität ist das Verhältnis der Permeanzen.

| Membran | J_{1-Buten} in m³_{N}/(m² h bar) | PS |
|---|---|---|
| 1 | 0,66 | 22,0 |
| 2 | 1,39 | 22,8 |
| 3 | 1,72 | 18,9 |
| 4 | 1,83 | 13,3 |
| 5 | 1,11 | 15,7 |
| 6 | 2,39 | 12,4 |
| 7 | 1,56 | 11,4 |
| 8 | 1,48 | 14,4 |
| 9 | 2,76 | 9,2 |

Aus den Ergebnissen ist ersichtlich, dass sich durch Zugabe von verschiedenen Zuschlägen die Transporteigenschaften der Zeolith-Membranen gezielt beeinflussen lassen. Dabei ist eine sehr große Steigerung der 1-Buten Permeanzen erreichbar, wobei kein oder nur ein geringer Verlust an Selektivität in Kauf genommen werden muss.

### e) Pervaporationsversuche:

Für Versuche zur Pervaporation wurde Membran 3 in einem Testmodul platziert. Ein Gemisch aus Ethanol und Wasser mit einem Anteil von 5 Gew.-% Ethanol wurde der Membran mit einer Temperatur von 40°C flüssig zugeführt. Der Permeatdruck betrug 13 mbar. Die Messungen ergaben einen Pertmeatfluss von 0,8 kg/(m² h). Die Ethanolkonzentration im Permeat betrug 68 Gew.-%.

## Patentansprüche

1. Verfahren zur Herstellung einer Komposit-Membran, wobei durch Hydrothermalsynthese auf einem porösen Träger eine oder mehrere, einen Zeolith vom MFI-Typ enthaltende, mikroporöse Trennschichten erzeugt werden, **dadurch gekennzeichnet, dass** der Syntheselösung für die Hydrothermalsynthese ein oder mehrere Zuschläge aus der Gruppe lineare (C₁-C₄)-Alkohole, Ammoniak, primäre, sekundäre und tertiäre Amine mit jeweils (C₁-C₄)-Alkylresten, Ethanolamin und (C₃-C₄)-Ketone zugegeben werden, und die Syntheselösung die folgenden molaren Bestandteile aufweist, wobei A für den Zuschlag steht:
SiO₂/Al₂O₃/Na₂O/TPAOH/TPABr/H₂O/A = 1/5x10-5 bis 5x10-2 / 0 bis 0,2 / 0 bis 0,1 / 0 bis 0,1 / (10-y) bis (100-y) / y
mit 0 < y ≤ 50,
wobei das molare Verhältnis von H₂O und Zuschlag A in der Syntheselösung 100-1: 1 beträgt.

2. Verfahren gemäß Anspruch 1, **dadurch gekennzeichnet, dass** als Zuschlag ein oder mehrere Verbindungen aus der Gruppe Methanol, Ethanol, n-Propanol, n-Butanol, primäre Amine mit (C₁-C₄)-Alkylgruppen, Ammoniak und Aceton zugegeben werden.

3. Verfahren nach Anspruch 2, **dadurch gekennzeichnet, dass** als Zuschlag ein oder mehrere Verbindungen aus der Gruppe Methanol, Ethanol, n-Propanol, und n-Propylamin zugegeben werden.

4. Verfahren nach Anspruch 1, **dadurch gekennzeichnet, dass** y einen Wert von 15 bis 35 aufweist.

5. Verfahren nach einem der Ansprüche 1 bis 4, **dadurch gekennzeichnet, dass** mehrere Zeolithschichten auf den Träger aufgebracht werden.

6. Komposit-Membran, erhältlich nach dem Verfahren gemäß einem der Ansprüche 1 bis 5.

7. Verwendung einer Komposit-Membran, erhältlich nach dem Verfahren gemäß einem der Ansprüche 1 bis 5, zur Stofftrennung durch Dampfpermeation, Gaspermeation oder Pervaporation und für die Filtration von Flüssigkeiten.

8. Verwendung nach Anspruch 7, zur Trennung von Butan/Isobutan oder Buten/Isobuten-Gemischen.

9. Verwendung nach Anspruch 7, zur Trennung von Ethanol/Wasser-Gemischen.

10. Verwendung nach Anspruch 7, zur Trennung von Isopropanol/Wasser-Gemischen.

## Claims

1. A process for the production of a composite membrane, one or more microporous separation layers comprising a zeolite of the MFI type being produced by hydrothermal synthesis on a porous substrate, wherein one or more additives from the group consisting of linear (C₁-C₄)-alcohols, ammonia, primary, secondary and tertiary amines having in each case (C₁-C₄) -alkyl radicals, ethanolamine and (C₃-C₄)-ketones are added to the synthesis solution for the hydrothermal synthesis, and the synthesis solution has the following molar constituents, A being the additive:
SiO₂/Al₂O₃/Na₂O/TPAOH/TPABr/H₂O/A = 1/5x10-5 to 5x10-2 / from 0 to 0.2 / from 0 to 0.1 / from 0 to 0.1 / (10-y) to (100-y) / y
with 0 < y ≤ 50,
where the molar ratio of H₂O and additive A in the synthesis solution is 100-1 : 1.

2. The process according to claim 1, wherein one or more compounds from the group consisting of methanol, ethanol, n-propanol, n-butanol, primary amines having (C₁-C₄)-alkyl groups, ammonia and acetone are added as the additive.

3. The process according to claim 2, wherein one or more compounds from the group consisting of methanol, ethanol, n-propanol and n-propylamine are added as the additive.

4. The process according to claim 1, wherein y has a value of from 15 to 35.

5. The process according to any of claims 1 to 4, wherein a plurality of zeolite layers is applied to the substrate.

6. A composite membrane obtainable by the process according to any of claims 1 to 5.

7. The use of a composite membrane obtainable by the process according to any of claims 1 to 5 for separating substances by vapor permeation, gas permeation or pervaporation and for the filtration of liquids.

8. The use according to claim 7, for the separation of butane/isobutane or butene/isobutene mixtures.

9. The use according to claim 7, for the separation of ethanol/water mixtures.

10. The use according to claim 7, for the separation of isopropanol/water mixtures.

## Revendications

1. Procédé de fabrication d'une membrane composite, selon lequel une ou plusieurs couches de séparation microporeuses contenant une zéolithe de type MFI sont formées par synthèse hydrothermale sur un support poreux, **caractérisé en ce qu'**un ou plusieurs additifs du groupe constitué par les alcools linéaires en (C₁-C₄), l'ammoniac, les amines primaires, secondaires et tertiaires contenant à chaque fois des radicaux alkyle en (C₁-C₄), l'éthanolamine et les cétones en (C₃-C₄) sont ajoutés à la solution de synthèse pour la synthèse hydrothermale, et la solution de synthèse comprend les constituants molaires suivants, A représentant l'additif :
SiO₂/Al₂O₃/Na₂O/TPAOH/TPABr/H₂O/A = 1/5 x 10⁻⁵ à 5 x 10⁻²/0 à 0,2/0 à 0,1/0 à 0,1/(10-y) à (100-y)/y
avec 0 < y ≤ 50,
le rapport molaire entre H₂O et l'additif A dans la solution de synthèse étant de 100 à 1:1.

2. Procédé selon la revendication 1, **caractérisé en ce qu'**un ou plusieurs composés du groupe constitué par le méthanol, l'éthanol, le n-propanol, le n-butanol, les amines primaires contenant des groupes alkyle en (C₁-C₄), l'ammoniac et l'acétone sont ajoutés en tant qu'additif.

3. Procédé selon la revendication 2, **caractérisé en ce qu'**un ou plusieurs composés du groupe constitué par le méthanol, l'éthanol, le n-propanol et la n-propylamine sont ajoutés en tant qu'additif.

4. Procédé selon la revendication 1, **caractérisé en ce qu'**y présente une valeur allant de 15 à 35.

5. Procédé selon l'une quelconque des revendications 1 à 4, **caractérisé en ce que** plusieurs couches de zéolithe sont appliquées sur le support.

6. Membrane composite, pouvant être obtenue par le procédé selon l'une quelconque des revendications 1 à 5.

7. Utilisation d'une membrane composite, pouvant être obtenue par le procédé selon l'une quelconque des revendications 1 à 5, pour la séparation de matières par perméation de vapeur, perméation de gaz ou pervaporation et pour la filtration de liquides.

8. Utilisation selon la revendication 7, pour la séparation de mélanges butane/isobutane ou butène/isobutène.

9. Utilisation selon la revendication 7, pour la séparation de mélanges éthanol/eau.

10. Utilisation selon la revendication 7, pour la séparation de mélanges isopropanol/eau.
